# EUROPEAN PATENT APPLICATION

(11) **EP 3 771 338 A1**
(43) Date of publication of application: **03.02.2021**
(21) Application number: 19188858.5
(22) Date of filing: 29.07.2019
(51) Int. Cl.: A01N 43/40, C11D 3/20, A01N 25/10, A01N 25/34, A01N 25/30, A01P 1/00, A61K 8/34

(54) **ACIDIC ANTIMICROBIAL COMPOSITION**

(71) Applicant: The Procter & Gamble Company, Cincinnati, OH 45202 (US)
(72) Inventor: KONYA, Abigail Mary, Newcastle upon Tyne, NE12 9TS (GB); PEREZ-PRAT VINUESA, Eva Maria, Newcastle upon Tyne, NE12 9TS (GB)
(74) Representative: P&G Patent Belgium UK

(57) **Abstract**

An antimicrobial cleaning composition having a pH of from about 2 to about 4 wherein the composition comprises a bispyridinium alkane antimicrobial active, a surfactant and an acid.

## Description

### FIELD OF THE INVENTION

The present invention is in the field of antimicrobial compositions. In particular, it relates to an acidic composition comprising a bispyridinium alkane antimicrobial active.

### BACKGROUND OF THE INVENTION

Acidic surface cleaners are commonly used to remove limescale from surfaces. Limescale deposits are formed when water containing soluble ions evaporates from the surface and these ions deposit on the surface as salts, such as calcium carbonate. The limescale formation and deposition phenomenon is even more acute in places where water is particularly hard. The visible limescale deposits result in an unappealing aspect of the surfaces. Furthermore, limescale deposits are prone to combination with other types of soils, such as soap scum or grease, and can lead to the formation of limescale-soil mixture deposits (limescale-containing soils) that are difficult to remove and can promote bacteria colonization of the surface. It is therefore desirable to include actives with biocidal activity in the acidic hard surface cleaner to provide surface sanitization or disinfection, at the same time as removing the limescale, and limescale-soil deposits.

Antibacterial actives commonly used in hard surface cleaners are quaternary ammonium compounds, and biguanides such as polyhexamethylene biguanide (PHMB) and chlorohexidine. Biguanides perform best at pH 5 to 7 and therefore are not the best choice for acidic compositions. Quaternary ammonium compounds show efficacy at a broad range of pH values but tend to be deposited on the surface as a visible residue, which leaves the user with an impression that the treated surface has not been cleaned well as it appears streaky and with poor shine. In addition, quaternary ammonium compounds typically interact with the cleaning surfactants present in the composition. The result is that the antimicrobial efficacy of the composition is reduced, or else, higher levels of the antimicrobial active must be present. The use of high levels of quaternary ammonium antibacterial actives in cleaning products is not desirable because these compounds are toxic to the environment and can cause contact dermatitis, nasal irritation and eye damage. Another drawback is that their level is restricted in products to be used on food contact surfaces.

There is therefore the need for an acidic antibacterial cleaner that has a good environmental and human safety profile, that shows strong antibacterial efficacy even at low levels of antibacterial active, that shows good cleaning of limescale and limescale-containing soil deposits, and that leaves the surfaces shiny and without streaks.

### SUMMARY OF THE INVENTION

According to the first aspect of the present invention, there is provided an antimicrobial cleaning composition.

According to the second aspect of the invention there is provided an article treated with the composition of the first aspect of the invention. The article is in the form of a disposable or partially reusable substrate comprising one or more nonwoven layers. The article provides sanitization to surfaces, in particular hard surfaces. The article is sometimes herein referred to as "the article of the invention".

According to the third aspect of the present invention, there is provided a method of sanitizing a surface using the composition of the invention.

The elements of the composition of the invention described in relation to the first aspect of the invention apply *mutatis mutandis* to the other aspects of the invention.

### DETAILED DESCRIPTION OF THE INVENTION

The present invention encompasses an antimicrobial composition. The composition is acidic and has a pH of from about 2 to about 4. The composition comprises bis pyridinium alkane, preferably octenidine dihydrochloride. Preferably, the composition is substantially free of quaternary ammonium antimicrobial active. By "substantially free" of quaternary ammonium antimicrobial active is herein meant that the composition comprises less than 0.001% by weight of the composition of a quaternary ammonium antimicrobial active.

The composition of the invention is well suited to be used to treat hard surfaces and fabrics. The composition is preferably a liquid composition, more preferably an aqueous liquid composition comprising at least 50% by weight of the composition of water. The composition may comprise from 80% to 99.5% by weight of the total composition of water, preferably from 82% to 98% and more preferably from 85% to 97%.

All percentages, ratios and proportions used herein are by weight percent of the composition, unless otherwise specified. All average values are calculated "by weight" of the composition, unless otherwise expressly indicated. All ratios are calculated as a weight/weight level, unless otherwise specified.

All measurements are performed at 25°C unless otherwise specified.

Unless otherwise noted, all component or composition levels are in reference to the active portion of that component or composition, and are exclusive of impurities, for example, residual solvents or by-products, which may be present in commercially available sources of such components or compositions.

### Antimicrobial cleaning compositions

The composition of the invention is suitable to be used on any type of surfaces, including hard surfaces and fabrics. The composition can be applied to a hard surface by for example spraying the composition, followed by wiping the surface, preferably without rinsing. Alternatively, the composition could be applied by using a substrate, such as a wipe impregnated with the composition of the invention.

As used herein, the terms "microbe" or "microbial" should be interpreted to refer to any of the microscopic organisms studied by microbiologists or found in the use environment of a treated surface. Such organisms include, but are not limited to, bacteria and fungi as well as other single-celled organisms such as mould, mildew and algae. Viruses (enveloped and non-enveloped) and other infectious agents are also included in the term microbe.

"Antimicrobial" further should be understood to encompass both microbiocidal and microbiostatic properties. That is, the term includes microbe killing, leading to a reduction in number of microbes, as well as a retarding effect of microbial growth, wherein numbers may remain more or less constant (but nonetheless allowing for slight increase/ decrease).

For ease of discussion, this description uses the term antimicrobial to denote a broad-spectrum activity (e.g. against bacteria and fungi, or against bacteria and viruses). When speaking of efficacy against a particular microorganism or taxonomic rank, the more focused term will be used (e.g. antifungal to denote efficacy against fungal growth in particular). Using the above example, it should be understood that efficacy against fungi does not in any way preclude the possibility that the same antimicrobial composition may demonstrate efficacy against another class of microbes.

By "hard surface ", it is meant herein hard surfaces found in households, especially domestic households. Surfaces to be cleaned include kitchens and bathrooms, e.g., floors, walls, tiles, windows, cupboards, sinks, showers, shower plastified curtains, wash basins, WCs, fixtures and fittings and the like made of different materials like ceramic, vinyl, no-wax vinyl, linoleum, melamine, glass, steel, kitchen work surfaces, any plastics, plastified wood, metal or any painted or varnished or sealed surface and the like. Household hard surfaces also include household appliances including, but not limited to refrigerators, freezers, washing machines, automatic dryers, ovens, microwave ovens, dishwashers and so on. Such hard surfaces may be found both in private households as well as in commercial, institutional and industrial environments.

The composition of the invention is also suitable to be used on fabrics, either directly, by for example spraying the composition onto the fabric, or in a washing machine during the prewash, main wash or any of the rinses.

The liquid compositions herein are aqueous compositions, comprising at least 10% by weight of water, preferably from 50% to 98% and more preferably from 80% to 97% by weight of the composition of water.

The compositions of the present invention preferably can be non-thickened, or water like, having a viscosity of from 1 mPa.s to 20 Pa.s, or can be thickened, having a viscosity of from 50 Pa.s to 1200 Pa.s, more preferably 100 Pa.s to 800Pa.s, most preferably 200 Pa.s to 600 Pa.s when measured at 20°C with a AD1000 Advanced Rheometer from Atlas® shear rate 10 s⁻¹ with a coned spindle of 40mm with a cone angle 2° and a truncation of ±60µm.

The low pH of the composition helps with cleaning, especially limescale removal. The pH of the composition is from 2 to 4. Accordingly, the composition herein comprises an acid to adjust the pH. A preferred organic acid for use herein has a pKa of less than 6. Suitable organic acids are selected from the group consisting of: formic acid, acetic acid, benzoic acid, malonic acid, citric acid, maleic acid, fumaric acid, succinic acid, gluconic acid, glutaric acid, lactic acid, cinnamic acid, salicylic acid, oxalic acid, itaconic acid, glycolic acid, adipic acid and mixtures thereof. A suitable inorganic acid can be selected from the group consisting of: hydrochloric acid, sulphuric acid, phosphoric acid and mixtures thereof.

A typical level of such acids, when present, is from 0.001% to 10% by weight of the total composition, preferably from 0.002% to 3.0% and more preferably from 0.005% to 1.5 %.

### Antimicrobial agent

The composition of the invention includes bispyridinium alkanes, such as the ones described in GB1533952. The term bispyridinium alkane comprises the bis[4-(substituted-amino)-1-pyridinium] alkanes of the general formulae (I) or (II) 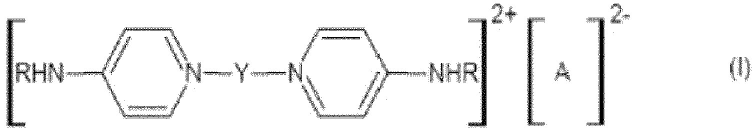 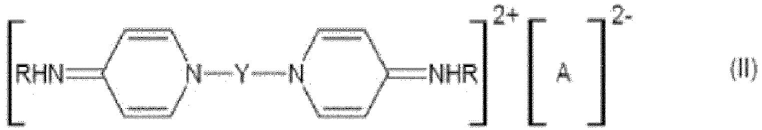 in which
Y is an alkylene or alkyl group having 4 to 18 carbon atoms,
R represents an alkyl group having 6 to 18 carbon atoms or a cycloalkyl group having 5 to 7 carbon atoms or a phenyl group with or without halogen substitution, and A is an anion or several anions.

A may be a monovalent, divalent or a polyvalent anion, for example chloride, bromide, phosphate or orthosilicate. A may also be an organic acid having the formula R4-COO∼, wherein R4 is hydrogen, hydroxyl, or C1-C40 alkyl.

Bispyridinium alkanes of the present invention comprise the various prototypes of the compounds of the formula (I) and (II) such as, for example, the ones disclosed in GB1533952 and DE19647692A1.

Other suitable bispyridinium alkanes comprise an organic acid salt of a bispyridine amine where the organic acid contains from about 4 to about 30 carbon atoms, such as, for example, the ones described in WO2014100807. Suitable organic acids include but are not limited to, carboxylic acids, such as (C1-C40) alkanecarboxylic acids which, for example, are unsubstituted or substituted by halogen, saturated or unsaturated dicarboxylic acids, such as hydroxycarboxylic acids, such as amino acids, such as (C1-C40) alkylsulfonic acids. Additional organic acids from which salts can be derived include, for example, acetic acid, propionic acid, phosphoric acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, glycyrrhizinic acid, salicylic acid, stearic acid, phosphonic acid, trifluoroacetic acid, cyanoacetic acid, 4-cyanobenzoic acid, 2-chlorobenzoic acid, 2-nitrobenzoic acid, phenoxyacetic acid, benzenesulfonic acid. Preferred are salts of stearate such as bispyridinium alkane distearate.

Preferred bispyridinium alkane is octenidine dihydrochloride (R = n-octyl, Y = n-decenyl; A = 2 × Cl, hereinbelow "octenidine" CAS number 70775-75-6).

The antimicrobial agent need only be present in germicidally effective amounts, which can be as little as 0.001% to less than 1% by weight of the composition. In more preferred compositions, the cleaning composition comprises the antimicrobial agent at a level of from from about 0.0025 to about 0.25%, more preferably from 0.005% to 0.15% by weight of the composition.

A germicidally effective amount of the antimicrobial agent typically results in at least a log 4 reduction of Staphylococcus aureus, using the method of EN13697 (Chemical Disinfectants Bactericidal Activity Testing), in 5 minutes.

### Quaternary ammonium compounds

The composition of the invention is substantially free of quaternary ammonium compounds.

Quaternary ammonium compounds include compounds of formula (A): 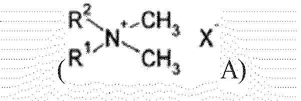 wherein R¹ and R² are each independently a straight chain, unsubstituted and uninterrupted C₈-C₁₂ alkyl group and X" is a halide anion such as chloride, bromide, fluoride, iodide or sulphonate, saccharinate, carbonate or bicarbonate, and benzalkonium compounds having the formula (B) 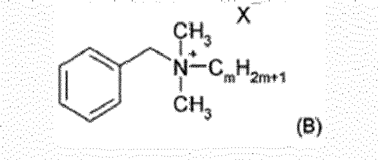 wherein m is from 8 to 18, and X" is a halide anion such as chloride, bromide, fluoride, iodide, sulphonate, saccharinate, carbonate or bicarbonate. This benzalkonium compounds usually comprise a mixture of Cs-Cis alkyl groups, particularly a mixture of straight chain, unsubstituted and uninterrupted alkyl groups such as n-C₈H₁₇ to n-C₁₈H₃₇, mainly n-C₁₂H₂₅ (dodecyl), n-C₁₄H₂₉ (tetradecyl), and n-C₁₆H₃₃(hexadecyl).

In the compounds of formula (A) each group R¹ and R² is independently a straight chain, unsubstituted, uninterrupted C₈₋₁₂ alkyl group, for example an alkyl group containing 8, 9, 10, 11 or 12 carbon atoms. The groups R¹ and R² may contain equal or different numbers of carbon atoms. Examples of quaternary ammonium compounds of formula (A) include di-n-decyldimethyl ammonium chloride, octyl decyl dimethyl ammonium chloride and dioctyl dimethyl ammonium chloride. Examples of quaternary ammonium compounds of formula (B) include N,N-benzyldimethyloctylammonium chloride, N,N-benzyldimethyldecylammonium chloride , N-dodecyl-N-benzyl-N,N-dimethylammonium chloride, and N-tetradecyl-N-benzyl-N,N-dimethylammonium chloride, N-hexadecyl-N,N-dimethyl-N-benzylammonium chloride.

### Surfactant

The composition of the invention preferably comprises surfactants, more preferably from 0.1 to 7%, especially from 0.5 to 5% by weight of the composition of surfactant. The surfactant contributes to the cleaning provided by the composition.

### Alcohol alkoxylated nonionic surfactants

Suitable alcohol alkoxylated nonionic surfactants are according to the formula RO-(A)nH, wherein: R is a primary C4 to C18, preferably a C6 to C16, more preferably a C6 to C14 branched or linear alkyl chain, or a C6 to C28 alkyl benzene chain; A is an ethoxy or propoxy or butoxy unit, or mixtures thereof, and wherein n is from 1 to 30, preferably from 1 to 15, more preferably from 3 to 12 even more preferably from 3 to 8. Preferred R chains for use herein are the C6 to C16 linear or branched alkyl chains.

Suitable branched alkoxylated alcohol may be selected from the group consisting of: C4-C10 alkyl branched alkoxylated alcohols, and mixtures thereof. The branched alkoxylated alcohol can be derived from the alkoxylation of C4-C10 alkyl branched alcohols selected form the group consisting of: C4-C10 primary mono-alcohols having one or more C1-C4 branching groups.

By C4-C10 primary mono-alcohol, it is meant that the main chain of the primary mono-alcohol has a total of from 4 to 10 carbon atoms. The C4-C10 primary mono-alcohol can be selected from the group consisting of: methyl butanol, ethyl butanol, methyl pentanol, ethyl pentanol, methyl hexanol, ethyl hexanol, propyl hexanol, dimethyl hexanol, trimethyl hexanol, methyl heptanol, ethyl heptanol, propyl heptanol, dimethyl heptanol, trimethyl heptanol, methyl octanol, ethyl octanol, propyl octanol, butyl octanol, dimethyl octanol, trimethyl octanol, methyl nonanol, ethyl nonanol, propyl nonanol, butyl nonanol, dimethyl nonanol, trimethyl nonanol and mixtures thereof.

The C4-C10 primary mono-alcohol can be selected from the group consisting of: ethyl hexanol, propyl hexanol, ethyl heptanol, propyl heptanol, ethyl octanol, propyl octanol, butyl octanol, ethyl nonanol, propyl nonanol, butyl nonanol, and mixtures thereof.

Preferably the C4-C10 primary mono-alcohol is selected from the group consisting of: ethyl hexanol, propyl hexanol, ethyl heptanol, propyl heptanol, and mixtures thereof.

The C4-C10 primary mono-alcohol is most preferably ethyl hexanol, and propyl heptanol.

In the branched alkoxylated alcohol, the one or more C1-C4 branching group can be substituted into the C4-C10 primary mono-alcohol at a C1 to C3 position, preferably at the C1 to C2 position, more preferably at the C2 position, as measured from the hydroxyl group of the starting alcohol.

The branched alkoxylated alcohol can comprise from 1 to 14, preferably from 2 to 7, more preferably from 4 to 6 ethoxylate units, and optionally from 1 to 9, preferably from 2 to 7, more preferably from 4 to 6 of propoxylate units.

The branched alkoxylated alcohol is preferably 2-ethyl hexan-1-ol ethoxylated to a degree of from 4 to 6, and propoxylated to a degree of from 4 to 6, more preferably, the alcohol is first propoxylated and then ethoxylated. Another preferred branched alkoxylated alcohols are 2-alkyl-1-alkanols such as alkoxylated C10 guerbet alcohols with 1 to 14, preferably from 2 to 7, more preferably from 3 to 6 ethoxylate or ethoxylate-propoxylate units.

Non-limiting examples of suitable branched alkoxylated alcohols are, for instance, Ecosurf® EH3, EH6, and EH9, commercially available from DOW, and Lutensol® XP alkoxylated Guerbet alcohols & Lutensol® XL ethoxylated Guerbet alcohols available from BASF.

Linear alcohol alkoxylated nonionic surfactants preferred herein are alkoxylated nonionic surfactants with a C8, C10, C12, mixtures of C8 to C10, mixtures of C10 to C12, mixtures of C9 to C11 linear alkyl chain and 10 or less ethoxylate units, preferably 3 to 8 ethoxylate units.

Non-limiting examples of suitable linear alkoxylated nonionic surfactants for use herein are Dobanol® 91-2.5 (R is a mixture of C9 and C11 alkyl chains, n is 2.5), Dobanol® 91-5 (R is a mixture of C9 to C11 alkyl chains, n is 5); Dobanol® 91-10 (R is a mixture of C9 to C11 alkyl chains, n is 10); Greenbentine DE60 (R is a C10 linear alkyl chain, n is 6); Marlipal 10-8 (R is a C10 linear alkyl chain, n is 8); Neodol 91-8 (R is a mixture of C9 to C11 alkyl chains, n is 8);; Lutensol ON30 (R is C10 linear alkyl chain, n is 3); Lutensol ON50 (R is C10 linear alkyl chain, n is 5); Lutensol ON70 (R is C10 linear alkyl chain, n is 7); Novel 610-3.5 (R is mixture of C6 to C10 linear alkyl chains, n is 3.5); Novel 810FD-5 (R is mixture of C8 to C10 linear alkyl chains, n is 5); Novel 10-4 (R is C10 linear alkyl chain, n is 4); Novel 1412-3 (R is mixture of C12 to C14 linear alkyl chains, n is 3); Lialethl® 11-5 (R is a C11 linear alkyl chain, n is 5); or mixtures thereof.

Preferred herein are alkoxylated non-ionic surfactants with a C8, C10, C12, mixtures of C8 to C10, mixtures of C10 to C12, mixtures of C9 to C11 linear alkyl chain with 8 or less ethoxylate units, preferably 3 to 8 ethoxylate units.

The alkoxylated nonionic surfactant may be a secondary alcohol ethoxylate such as for example the TergitolTM-15-S surfactants having the general formula shown below and commercially available by DOW 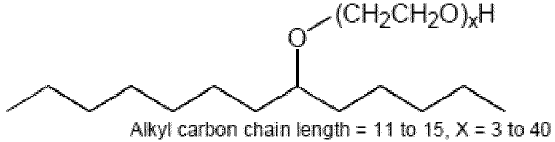 Tergitol 15-S surfactants

Preferred secondary alcohol ethoxylate surfactants have 3-9 EO units.

Another suitable alkoxylated nonionic surfactant is an alkyl ethoxy alkoxy alcohol, preferably wherein the alkoxy part of the molecule is propoxy, or butoxy, or propoxy-butoxy. More preferred alkyl ethoxy alkoxy alcohols are of formula (II): 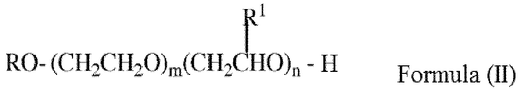 wherein:
R is a branched or unbranched alkyl group having 8 to 16 carbon atoms;
R1 is a branched or unbranched alkyl group having 1 to 5 carbon atoms;
n is from 1 to 10; and m is from 6 to 35.
R is preferably from 12 to 15, preferably 13 carbon atoms. R1 is preferably a branched alkyl group having from 1 to 2 carbon atoms. n is preferably 1 to 5. m is preferably from 8 to 25. Preferably, the weight average molecular weight of the ethoxylated alkoxylated nonionic surfactant of formula (II) is from 500 to 2000g/mol, more preferably from 600 to 1700 g/mol, most preferably 800 to 1500 g/mol.

The ethoxylated alkoxylated nonionic surfactant can be a polyoxyalkylene copolymer. The polyoxyalkylene copolymer can be a block-heteric ethoxylated alkoxylated nonionic surfactant, though block-block surfactants are preferred. Suitable polyoxyalkylene block copolymers include ethylene oxide/propylene oxide block polymers, of formula (III):

(EO)x(PO)y(EO)x,

or

(PO)x(EO)y(PO)x

wherein EO represents an ethylene oxide unit, PO represents a propylene oxide unit, and x and y are numbers detailing the average number of moles ethylene oxide and propylene oxide in each mole of product. Such materials tend to have higher molecular weights than most non-ionic surfactants, and as such can range between 1000 and 30000 g/mol, although the molecular weight should be above 2200 and preferably below 13000 to be in accordance with the invention. A preferred range for the molecular weight of the polymeric non-ionic surfactant is from 2400 to 11500 Daltons. BASF (Mount Olive, N.J.) manufactures a suitable set of derivatives and markets them under the Pluronic trademarks. Examples of these are Pluronic (trademark) F77, L62 and F88 which have the molecular weight of 6600, 2450 and 11400 g/mol respectively.

Other suitable ethoxylated alkoxylated nonionic surfactants are described in Chapter 7 of Surfactant Science and Technology, Third Edition, Wiley Press, ISBN 978-0-471-68024-6.

Most preferably the alkoxylated nonionic surfactant is selected from the group consisting of: C9-C11 EO8 alcohol ethoxylate (Neodol 91-8-Sasol), 2-propylheptyl EO8 alcohol alkoxylate (Lutensol XL89-BASF); 2-propylheptyl EO5 alcohol alkoxylate (Lutensol XL50-BASF); C10 EO5 alcohol ethoxylate (Lutensol ON 50-BASF); C10 EO7 alcohol ethoxylate (Lutensol ON 70-BASF); C8-C10 EO5 alcohol ethoxylate (Novel 810 FD5 Sasol); C10 EO4 alcohol ethoxylate (Novel 10-4 Sasol); Tergitol 15-S-3; Tergitol 15-S-5; Tergitol 15-S-7; and Ethyl hexanol PO5EO6 (Ecosurf EH6-Dow). These surfactants have surprisingly been found to potentiate the antibacterial efficacy of octenidine.

### Alkyl Polyglucosides

Alkyl polyglycosides are biodegradable nonionic surfactants which are well known in the art, and can be used in the compositions of the present invention. Suitable alkyl polyglycosides can have the general formula CnH2n+1O(C6H10O5)xH wherein n is preferably from 8 to 16, more preferably 8 to 14, and x is at least 1. Examples of suitable alkyl polyglucoside surfactants are the TRITON™ alkyl polyglucosides from Dow; Agnique PG, Disponil APG and Glucopon alkyl polyglucosides from BASF. Preferred alkyl polyglucoside surfactants are those where n is 8 to 12, more preferably 8 to 10, such as for example Triton™ CG50 (Dow). These surfactants have surprisingly been found to enhance the antimicrobial efficacy of octenidine.

### Amine Oxide

Suitable amine oxide surfactants include: R1R2R3NO wherein each of R1, R2 and R3 is independently a saturated or unsaturated, substituted or unsubstituted, linear or branched hydrocarbon chain having from 1 to 30 carbon atoms. Preferred amine oxide surfactants are amine oxides having the following formula: R1R2R3NO wherein R1 is a hydrocarbon chain comprising from 1 to 30 carbon atoms, preferably from 6 to 20, more preferably from 8 to 16 and wherein R2 and R3 are independently saturated or unsaturated, substituted or unsubstituted, linear or branched hydrocarbon chains comprising from 1 to 4 carbon atoms, preferably from 1 to 3 carbon atoms, and more preferably are methyl groups. R1 may be a saturated or unsaturated, substituted or unsubstituted linear or branched hydrocarbon chain.

Highly preferred amine oxides are C8 dimethyl amine oxide, C10 dimethyl amine oxide, C12 dimethyl amine oxide, C12-C14 dimethyl amine oxide, and C14 dimethyl amine oxide, and mixtures thereof. C8 dimethyl amine oxide is commercially available under the trade name Genaminox® OC from Clariant; C10 dimethyl amine oxide is commercially available under the trade name Genaminox® K-10 from Clariant; C12 dimethyl amine oxide is commercially available under the trade name Genaminox® LA from Clariant and of Empigen OB from Huntsman; C14 amine oxide is commercially available under the trade name of Empigen OH 25 from Huntsman. Other suitable amine oxide surfactants are cocoyldiethoxy amine oxide available under the trade name of Genaminox CHE from Clariant, and cocamydopropyl amine oxide commercially available under the trade name of Empigen OS/A from Huntsman. Particularly preferred amine oxide surfactants are C10 dimethyl amine oxide such as Genaminox K-10, and C12 dimethyl amine oxide such as Empigen OB These surfactants have surprisingly been found to greatly enhance octenidine antibacterial efficacy.

### Alkyl glucamide surfactants

The composition of the invention may comprise an alkyl glucamide surfactant. Glucamide surfactants are non- ionic surfactants in which the hydrophilic moiety (an amino-sugar derivative) and the hydrophobic moiety (a fatty acid) are linked via amide bonds. This results in a chemical linkage, which is highly stable under alkaline conditions. Particularly preferred alkyl glucamide surfactants are N-alkyl-N-acylglucamides of the formula (III): 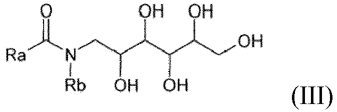 Wherein Ra is a linear or branched, saturated or unsaturated hydrocarbyl group having 6 to 22 carbon atoms, and Rb is a C1-C4 alkyl group. Particularly preferably, Rb in formula (I) is a methyl group. Non-limiting examples of these glucamide surfactants are: N-octanoyl-N-methylglucamide, N-nonanoyl-N-methylglucamide, N-decanoyl-N-methylglucamide, N-dodecanoyl-N-methylglucamide, N-cocoyl-N-methylglucamide, (available under the trade name of GlucoPure Foam by from Clariant), N-lauroyl/myristoyl-N-methylglucamide, (available under the trade name of GlucoPure Deg by from Clariant), and N-octanoyl/decanoyl-N-methylglucaminemethylglucamide, (available under the trade name of GlucoPure Wet by Clariant).

### Alkyl glucamine surfactants

The compositions of the invention may comprise an alkyl glucamine surfactant. These surfactants are described in EP16184415 and US20190055496.
Zwitterionic and amphoteric surfactants:
The hard surface cleaning composition may comprise an amphoteric surfactant, a zwitterionic surfactant, and mixtures thereof. Suitable zwitterionic surfactants typically contain both cationic and anionic groups in substantially equivalent proportions so as to be electrically neutral at the pH of use, and are well known in the art. Some common examples of zwitterionic surfactants are described in US. Pat. Nos. 2,082,275, 2,702,279 and 2,255,082.

Suitable zwitteronic surfactants include betaines such alkyl betaines, alkylamidobetaine, amidazoliniumbetaine, sulfobetaine (INCI Sultaines) as well as the phosphobetaine.

Suitable betaines are the alkyl betaines of the formula (Ia), the alkyl amido betaine of the formula (Ib), the sulfo betaines of the formula (Ic) and the amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 is a saturated or unsaturated C6-C22 alkyl residue, preferably C8-C18 alkyl residue. Particularly preferred are betaines of the formula (Ia) such as for example N-alkyl-N-dimethyl betaine like the one sold under the name of Empigen® BB by Huntsman, and (Id) such as for example cocoamidopropyl hydroxysultaine sold under the name of Mackam ®50 SB by Solvay Novacare.

Other examples of suitable betaines and sulfobetaine are the following designated in accordance with [INCI]: Almondamidopropyl of betaines, Apricotamidopropyl betaines, Avocadamidopropyl of betaines, Babassuamidopropyl of betaines, Behenamidopropyl betaines, Behenyl of betaines, betaines, Canolamidopropyl betaines, Capryl/Capramidopropyl betaines, Carnitine, Cetyl of betaines, Cocamidoethyl of betaines, Cocamidopropyl betaines, , Coco betaines, Coco Hydroxysultaine, Coco/Oleamidopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucamidopropyl Hydroxysultaine, Hydrogenated Tallow of betaines, Isostearam idopropyl betaines, Lauramidopropyl betaines, Lauryl of betaines, Lauryl Hydroxysultaine, Lauryl Sultaine, Milkamidopropyl betaines, Minkamidopropyl of betaines, Myristamidopropyl betaines, Myristyl of betaines, Oleamidopropyl betaines, Oleamidopropyl Hydroxysultaine, Oleyl of betaines, Olivamidopropyl of betaines, Palmam idopropyl betaines, Palmitan idopropyl betaines, Palmitoyl Carnitine, Palm Kernelamidopropyl betaines, Polytetrafluoroethylene Acetoxypropyl of betaines, Ricinoleamidopropyl betaines, Sesam idopropyl betaines, Soyamidopropyl betaines, Stearamidopropyl betaines, Stearyl of betaines, Tallowamidopropyl betaines, Tallowamidopropyl Hydroxysultaine, Tallow of betaines, Tallow Dihydroxyethyl of betaines, Undecylenamidopropyl betaines and Wheat Germamidopropyl betaines.

If the composition comprises a zwitterionic surfactant, it is preferably a betaine of the formula Ia such as for example N-alkyl-N-dimethyl betaine like the one sold under the trade name of Empigen® BB by Huntsman., or an alkylamidopropyl hydroxysultaine such as for example Mackam®50 SB (Solvay Novacare) It has been found these betaines greatly increase the antibacterial efficacy of octenidine hydrochloride.

Amphoteric surfactants can be either cationic or anionic depending upon the pH of the composition. Suitable amphoteric surfactants include dodecylbeta-alanine, N-alkyltaurines such as the one prepared by reacting dodecylamine with sodium isethionate, as taught in US. Pat. No. 2,658,072, N-higher alkylaspartic acids such as those taught in U.S. Pat. No. 2,438,091. Other suitable amphoteric surfactants are the products sold under the trade name Miranol by Solvay-Novecare such as, for example, sodium lauroamphoacetate (Miranol Ultra L-32E), sodium stearoampho acetate (Miranol DM), disodium cocoamphodiacetate (Miranol C2m Conc NP), disodium lauroamphodiacetate (Miranol BM Conc), disodium capryloampho dipropionate (Miranol JBS), sodium mixed C8 amphocarboxylate (Miranol JEM Conc), and sodium capryloampho hydroxypropyl sulfonate (Miranol JS). Other non-limiting examples of suitable amphoteric surfactants are disodium capryloamphodiacetate (Mackam 2CY 75-Solvay Novecare), octyliminodipropionate (Ampholak YJH40-Akzo Nobel), sodium lauriminodipropionate (Mirataine H2C-HA-Solvay Novecare), and sodium lauroamphohydroxypropylsulfonate (Mackam LS- Solvay Novecare). It has been found that these amphoteric surfactants do not impact negatively the antimicrobial efficacy of octenidine hydrochloride.

Other suitable additional surfactants can be found in McCutcheon's Detergents and Emulsifiers, North American Ed. 1980.

### Anionic surfactants

If anionic surfactant is present, it is preferably present at low levels, i.e. below 0.05% by weight of the composition.

Particularly preferred surfactants for use herein include nonionic surfactants, in particular branched alcohol alkoxylates, more in particular 2-ethyl hexan-1-ol ethoxylated to a degree of from 4 to 6, and propoxylated to a degree of from 4 to 6, more preferably, the alcohol is first propoxylated and then ethoxylated, and 2-alkyl-1-alkanols such as alkoxylated C₁₀ guerbet alcohols with 1 to 14, preferably 2 to 8, more preferably 3 to 6 ethoxylate or ethoxylate-propoxylate units. Other particularly preferred non-ionic surfactants include linear alcohol alkoxylated nonionic surfactants with C₈, C₁₀, C₁₂, mixtures of C₈ to C₁₀, mixtures of C₁₀ to C₁₂, mixtures of C₉ to C₁₁ linear alkyl chain and 10 or less ethoxylate units, preferably 3 to 8 ethoxylate units. Most preferably the alkoxylated nonionic surfactant is selected from the group consisting of: C9-C11 EO8 alcohol ethoxylate (Neodol 91-8-Sasol), 2-propylheptyl EO8 alcohol alkoxylate (Lutensol XL89-BASF); 2-propylheptyl EO5 alcohol alkoxylate (Lutensol XL50-BASF); C10 EO5 alcohol ethoxylate (Lutensol ON 50-BASF); C10 EO7 alcohol ethoxylate (Lutensol ON 70-BASF); C8-C10 EO5 alcohol ethoxylate (Novel 810 FD5 Sasol); C10 EO4 alcohol ethoxylate (Novel 10-4 Sasol); Tergitol 15-S-3; Tergitol 15-S-5; Tergitol 15-S-7; and Ethyl hexanol PO5EO6 (Ecosurf EH6-Dow). These surfactants have surprisingly been found to potentiate the antibacterial efficacy of Octenidineoctenidine.

Other particularly preferred surfactants for use here in include linear amine oxide surfactants, in particular C₈-C₁₂ dimethyl amine oxide, more in particular C₁₀ dimethyl amine oxide; and C12 dimethyl amine oxide; alkyldimethylbetaine surfactants, more in particular N,N-Dimethyl-N-dodecylglycine betaine (Empigen BB-Huntsman); alkylamidopropyl hydroxysultaine surfactants (Mackam® 50 SB); alkyl glucamide surfactants such as N-alkyl-N-acylglucamide preferably N-decanoyl-N-methylglucamine, and the alkyl glucamide surfactants sold under the name of GlucoPure®, GlucoTain®, and GlucoWet® by Clariant; alkylpolyglucoside surfactants, more in particular C₈ to C₁₂ alkyl polyglucosides, more preferably C₈ to C₁₀ alkyl polyglucosides such as for example Triton® CG50 (Dow)

These surfactants improve the antimicrobial activity of the bispyridinium alkane, in particular the antimicrobial activity of octenidine dihydrochloride.

### Optional ingredients

### Chelating agent

The antimicrobial cleaning composition can comprise a chelating agent. Suitable chelating agents, in combination with the surfactant system, improve the shine benefit. Chelating agent can be incorporated into the compositions in amounts ranging from 0.05% to 5.0% by weight of the composition, preferably from 0.1% to 3.0%, more preferably from 0.2% to 2.0% and most preferably from 0.2% to 0.4%.

Suitable phosphonate chelating agents include ethylene diamine tetra methylene phosphonates, and diethylene triamine penta methylene phosphonates (DTPMP), and can be present either in their acid form or as salts.

A preferred biodegradable chelating agent for use herein is ethylene diamine N,N'-disuccinic acid, or alkali metal, or alkaline earth, ammonium or substitutes ammonium salts thereof or mixtures thereof, for instance, as described in US patent 4, 704, 233. A more preferred biodegradable chelating agent is L-glutamic acid N,N-diacetic acid (GLDA) commercially available under tradename Dissolvine 47S from Akzo Nobel.

Suitable amino carboxylates include ethylene diamine tetra acetates, diethylene triamine pentaacetates, diethylene triamine pentaacetate (DTPA), N- hydroxyethylethylenediamine triacetates, nitrilotriacetates, ethylenediamine tetrapropionates, triethylenetetraaminehexa-acetates, ethanoldiglycines, and methyl glycine diacetic acid (MGDA), both in their acid form, or in their alkali metal, ammonium, and substituted ammonium salt forms. Particularly suitable amino carboxylate to be used herein is propylene diamine tetracetic acid (PDTA) which is, for instance, commercially available from BASF under the trade name Trilon FS® and methyl glycine di-acetic acid (MGDA). Most preferred aminocarboxylate used herein is diethylene triamine pentaacetate (DTPA) from BASF. Further carboxylate chelating agents for use herein include salicylic acid, aspartic acid, glutamic acid, glycine, malonic acid or mixtures thereof.

Suitable polycarboxylates include itaconic acid and sodium polyitaconate which is, for instance, commercially available from Itaconix under the trade name of Itaconix® DSP 2K™, and Itaconix® CHT121™.

### Polymers

The antimicrobial cleaning composition may comprise an additional polymer. It has been found that the presence of a specific polymer as described herein, when present, allows further improvement of the grease removal performance of the liquid composition due to the specific sudsing/foaming characteristics they provide to the composition.

The polymer can be selected from the group consisting of: a vinylpyrrolidone homopolymer (PVP); a polyethyleneglycol dimethylether (DM-PEG); a vinylpyrrolidone/dialkylaminoalkyl acrylate or methacrylate copolymers; a polystyrenesulphonate polymer (PSS); a poly vinyl pyridine-N-oxide (PVNO); a polyvinylpyrrolidone/ vinylimidazole copolymer (PVP-VI); a polyvinylpyrrolidone/ polyacrylic acid copolymer (PVP-AA); a polyvinylpyrrolidone/ vinylacetate copolymer (PVP-VA); a polyacrylic polymer or polyacrylicmaleic copolymer; and a polyacrylic or polyacrylic maleic phosphono end group copolymer; and mixtures thereof.

Typically, the antimicrobial hard surface cleaning composition may comprise from 0.005% to 5.0% by weight of the total composition of said polymer, preferably from 0.10% to 4.0%, more preferably from 0.1% to 3.0% and most preferably from 0.20% to 1.0%.

### Solvent

The liquid compositions of the present invention may comprise a solvent or mixtures thereof as a preferred optional ingredient. Suitable solvent is selected from the group consisting of: ethers and diethers having from 3 to 14 carbon atoms; glycols (such as propylene glycol), or alkoxylated glycols; alkoxylated aromatic alcohols; aromatic alcohols; alkoxylated aliphatic alcohols; aliphatic alcohols; C₈-C₁₄ alkyl and cycloalkyl hydrocarbons and halohydrocarbons; C₆-C₁₆ glycol ethers; terpenes; and mixtures thereof. Ethers such as n-butoxypropanol and glycol ethers such as dipropylene glycol n-butyl ether are particularly preferred.

When present, the solvent can be present at a level of from 0.1% to 10%, or 0.2% to 5%, or 0.5% to 3% by weight of the composition.

### Thickener

The composition of the invention can further comprise a thickener. A thickener provides a higher viscosity cleaning composition which gives longer contact time and therefore more time for the composition to penetrate into the greasy soil and/or particulated greasy soil to improve cleaning effectiveness. A thickener can also improve product stability.

Suitable thickeners herein include polyacrylate based polymers, preferably hydrophobically modified polyacrylate polymers; amide polymers; hydroxyl ethyl cellulose, preferably hydrophobically modified hydroxyl ethyl cellulose, xanthan gum, hydrogenated castor oil (HCO) and mixtures thereof. Other suitable thickeners are amide polymers. Suitable amide polymers are polymerized fatty acid-based polyamides, as described in US20030162938A1.

The most preferred thickener used herein are hydrophobic alkali swellable emulsion (HASE) thickeners. As such, the antimicrobial hard surface cleaning composition preferably comprises from 0.1% to 10.0% by weight of the total composition of said thickener, preferably from 0.2% to 5.0%, more preferably from 0.2% to 2.5% and most preferably from 0.2% to 2.0%.

An increased viscosity, especially low shear viscosity, provides longer contact time, especially on inclined surfaces, and therefore improved penetration of greasy soil and/or particulated greasy soil. As a result, an increased viscosity improves cleaning and antimicrobial efficacy, especially when applied neat to the surface to be treated. Moreover, a high low shear viscosity improves the phase stability of the liquid cleaning composition, and especially improves the stability of the copolymer in compositions in the antimicrobial hard surface cleaning composition. Hence, preferably, the antimicrobial hard surface cleaning composition, comprising a thickener, has a viscosity of from 50 Pa.s to 1200 Pa.s, more preferably 100 Pa.s to 800Pa.s, most preferably 200 Pa.s to 600 Pa.s, at 20°C when measured with a AD1000 Advanced Rheometer from Atlas® shear rate 10 s⁻¹ with a coned spindle of 40mm with a cone angle 2° and a truncation of ±60µm.

The hydrophobically modified alkali swellable emulsion (HASE) comprises a thickening polymer , the thickening polymer comprising the following monomers:
(a) greater than 10 mol% of a carboxylic acid containing monomer;
(b) less than 90 mol% of an alkyl (meth)acrylate monomer;
(c) 0 to 3 wt%, preferably 0.1 to 2%, more preferably 0.5 to 2% of an associative monomer according to formula (I) or formula (II):

   R₁-CH=CH-COO-(CH₂CH₂O)ₙ-R₂ formula (I)

   R₁-CH=CH-R₃-NH-COO-(CH₂CH₂O)ₙ-R₂ formula (II)

   in which:
   (i) R₁ is H, C or COOH;
   (ii) R₂ is a C8-C30 alkyl chain, preferably aliphatic, preferably saturated, preferably linear;
   (iii) n is an integer between 2 and 150, preferably between 2 and 50, more preferably between 8 and 30, most preferably between 10 and 26; and
   (iv) R₃ is a C1-12 alkyl chain, which can be linear, branched, aromatic or combinations thereof;

For improved transparency, the carboxylic acid containing monomer is preferably present at a level greater than 20 mol%, more preferably 25 mol%, even more preferably greater than 35 mol% of the thickening polymer. The carboxylic acid containing monomer can be selected from the group consisting of: acrylic acid, methacrylic acid, itaconic acid or maleic acid, and mixtures thereof. For improved thickening, the carboxylic acid containing monomer is preferably present at the level of less than 80 mol%, more preferably less than 75 mol%, even more preferably 65 mol%.

For improved transparency, the alkyl (meth)acrylate monomer is more preferably present at a level of less than 75 mol%, more preferably less than 65 mol% of the thickening polymer. Any suitable alkyl chain can be used, though C₁-C₈ is preferred. In more preferred embodiments, the alkyl chain is ethyl (C₂) or butyl (C₄). The alkyl chain can be attached to the (meth)acrylate group by any suitable means, though ester bonds are preferred. For improved thickening the alkyl (meth)acrylate monomer is more preferably present at a level of greater than 10 mol%, more preferably greater than 30 mol%.

The monomers of the thickening polymer sum up to 100 mol%.

The thickening polymer is preferably not crosslinked. The monomers can be randomly distributed or distributed in blocks, though random is preferred for improved thickening.

Compositions which comprise a HASE thickener, in which the thickening polymer comprises greater than 20 mol% of a carboxylic acid containing monomer, less than 80 mol% of an alkyl (meth)acrylate monomer, and 0 to 3 mol%, preferably 0.1 to 2 mol%, more preferably 0.5 mol% to 2 mol% of an associative monomer according to formula (I) or formula (II), and particularly effective at maintaining the antimicrobial effect of the antimicrobial agent.

The thickening polymer preferably has a weight average molecular weight of from 50,000 Da to 2,000,000 Da, more preferably from 100,000 Da to 1,000,000 Da, most preferably from 300,000 Da to 600,000 Da.

### Other optional ingredients

The composition of the invention may comprise a variety of other optional ingredients depending on the technical benefit aimed for and the surface treated. Suitable optional ingredients for use herein include perfume, builders, other polymers, buffers, hydrotropes, colorants, stabilisers, radical scavengers, abrasives, soil suspenders, brighteners, anti-dusting agents, dispersants, dye transfer inhibitors, pigments, silicones and/or dyes.

### Wipe

The present invention also relates to an article treated with the composition of the invention. The article is preferably a wipe. Suitable wipes can be fibrous. Suitable fibrous wipes can comprise polymeric fibres, cellulose fibres, and combinations thereof. Suitable cellulose-based wipes include kitchen wipes, and the like. Suitable polymeric fibres include polyethylene, polyester, and the like. Polymeric fibres can be spun-bonded to form the wipe. Methods for preparing thermally bonded fibrous materials are described in U.S. application Ser. No. 08/479,096 (see especially pages 16-20) and U.S. Pat. No. 5,549,589 (see especially Columns 9 to 10). Suitable pads include foams and the like, such as HIPE-derived hydrophilic, polymeric foam. Such foams and methods for their preparation are described in U.S. Pat. No. 5,550,167; and U.S. patent application Ser. No. 08/370,695.

The load factor is defined as the weight ratio of antimicrobial solution to nonwoven substrate is preferably from about 3X to about 10X. Preferably, the load factor is between 4X and 8X, or from 4.5X to 7.5X, or from 5X to 7X. It is found that higher load factors for the pre-moistened wipes of the invention are preferable since they help increase product mileage.

### Method of cleaning a surface

The composition of the invention is particularly suited for cleaning surfaces selected from the group consisting of: ceramic, enamel, stainless steel, Inox®, Formica®, vinyl, no-wax vinyl, linoleum, melamine, glass, plastics and plastified wood, and combinations thereof. In particular, the compositions are particularly suited for reducing the microbial population, while leaving surfaces clean, shiny and grease free.

The composition of the invention can be used neat or can be achieved by diluting with water a concentrated composition prior to applying to the surface. In preferred methods, the composition is applied neat, more preferably, the hard surface cleaning composition is sprayed onto the hard surface. The composition can be applied by any suitable means, including using a mop, sponge, cloth, paper towel, or other suitable implement.

The hard surface may be rinsed, preferably with clean water, in an optional further step, and also as a further step, wiped, such as with a cloth or a paper towel.

In another preferred embodiment of the present invention said method of cleaning a hard surface includes the steps of applying, preferably spraying, said liquid composition onto said hard surface, leaving said liquid composition to act onto said surface for a period of time with or without applying mechanical action, and optionally removing said liquid composition, preferably removing said liquid composition by rinsing said hard surface with water and/or wiping said hard surface with an appropriate implement, e.g., a sponge, a paper or cloth towel and the like. Such compositions are often referred to as "ready-to-use" compositions. In preferred methods, the hard surface is not rinsed after application of the antimicrobial hard surface cleaning composition.

It is believed that acidic antimicrobial compositions comprising specific surfactants, and a bispyridinium alkane antimicrobial agent such as octenidine deliver very good antimicrobial efficacy at very low octenidine levels. This strong efficacy results from the careful selection of the surfactant type and level to achieve a synergistic interaction between the surfactant and the antimicrobial active. As a result, the antimicrobial efficacy of the antimicrobial agent in the antimicrobial composition is improved. Indeed, it has been found that the antimicrobial hard surface cleaning compositions of the present invention exhibit improved antimicrobial efficacy, good grease cleaning and streak-free shine.

### METHODS

### A) pH measurement

The pH is measured on the neat composition, at 25°C, using a pH meter with compatible gel-filled pH probe (such as Sartarius PT-10P meter with Toledo probe part number 52 000 100), calibrated according to the instruction manual.

### B) Antibacterial Efficacy (Minimum Biocidal Concentration in suspension)

The antimicrobial efficacy of the antimicrobial agent in the composition is determined by measuring its Minimum Biocidal Concentration (MBC). The MBC is defined as the lowest absolute concentration of the particular antimicrobial active which provides complete kill (zero bacterial growth). The MBC of the compositions herein is determined against the bacterium, *Staphylococcus aureus* (*Saureus* - ATCC #6538), a gram positive bacterium, These microorganisms are representative of natural contaminants in many consumer and industrial applications. The bacteria inoculum is prepared by transferring several colonies from a Tryptone Soy Agar (TSA) plate to a saline solution (0.85% NaCl) comprising 5% of horse serum as additional soil load when indicated, the bacteria concentration in this saline solution is determined by measuring the % Transmittance at 425 nm and adjusted by either adding more bacteria or more saline solution until the %Transmittance at 425 nm is between 23-25% which corresponds to a bacteria concentration of 10⁸CFU/ml.

The antimicrobial agent was added to the composition at a level of 1000 ppm, 500 ppm, or 200 ppm, or 100 ppm or 50 ppm. 200 µL of the antimicrobial hard surface cleaning composition was dosed into one well of row A of a 96 well microtiter plate. Each subsequent well (rows B to G) were dosed with 100 µL of the same hard surface cleaning composition, without the addition of the antimicrobial agent. 100 µL of the antimicrobial hard surface cleaning composition was transferred from row A to row B and mixed. 100 µL of the composition was then transferred from row B to row C and mixed, and the process repeated to row G. As such, the concentration of the antimicrobial agent underwent two-fold dilution in adjacent wells, while the concentration of the other actives in the hard surface cleaning composition remained constant across all the wells in the same column.

10 µL of the 10⁸CFU/ml bacteria suspension in saline was added to wells A to F of the microtiter plate with row G kept as a nil bacteria control. The final volume in each well was 110 µL, except for row G which comprised 100 µL of the hard surface cleaning composition and no bacteria suspension. Bacterial inoculation to each column was staggered by 30 seconds to allow for equal incubation times in all wells so that the contact time between the bacteria and the antimicrobial active for all samples was 6 mins. After this contact time, 10µL of each test solution was transferred to 90µL of neutraliser solution (Modified Letheen Broth + 1.5% Polysorbate 80, supplied by BioMérieux) to stop the antimicrobial action of the antimicrobial active matching the stagger of the inoculation. 2µL of this solution was plated onto a TSA plate matching the stagger of the inoculation so that all samples are exposed to the neutralizer for the same period of time. The plate is incubated at 32.5°C for 48h for *S.aureus.* MBC concentration is taken as the lowest concentration of the antimicrobial active at which no visible colony growth is observed on the TSA plate.

### EXAMPLES

### Comparison of antibacterial efficacy of bispyridinium alkane vs biocidal quaternary ammonium compound in acidic compositions comprising different surfactants

The antibacterial efficacy of the bispyridinium alkane octenidine hydrochloride was compared to that of the quaternary ammonium compound n-alkyl dimethyl benzyl ammonium chloride/n-alkyl dimethyl ethylbenzyl ammonium chloride (BTC®2125-Stepan) in acidic compositions comprising 1% of the indicated surfactant. Compositions were acidified using either an inorganic acid (hydrochloric acid) or an organic acid (citric acid). The minimal biocidal concentration (MBC) in suspension of each antibacterial active against a gram positive bacterium (*Staphylococcus aureus*) was measured in the presence of 5% horse serum to mimic stressed soiled conditions. Contact time was 6 minutes except for the composition with 1% of dodecyldimethyl amine oxide that was 30 seconds. Results are shown in Table 1.

| **Table 1: MBC in suspension of bispyridinium alkane vs quaternary ammonium biocide in acidic compositions comprising 1% of the indicated surfactant** | | | | |
|---|---|---|---|---|
| Surfactant at 1 % w/v concentration | Acid | pH | Octenidine MBC (ppm) | BTC®2125 MBC (ppm) |
| Dodecyl dimethyl amine oxide (1) | Citric | 3.88 | 62.5 | 500 |
| C10 EO5 alcohol ethoxylate (2) | HCl | 3.86 | Less than 31 | 62.5 |
| 2-ethylhexyl PO5EO6 alkylalkoxylate (3) | HCl | 3.87 | Less than 31 | 250 |
| D-glucopyranose oligomeric decyl octyl glucoside (4) | Citric | 3.83 | 62.5 | 250 |
| C9-C11 alcohol EO8 (5) | HCl | 3.86 | 62.5 | 500 |
| Cocoamidopropyl hydroxysultaine (6) | HCl | 3.83 | 62.5 | 500 |

| | | | | |
|---|---|---|---|---|
| (1) Empigem OB - Huntsman (2) Lutensol ON 50-BASF (3) Ecosurf EH6 -Dow (4) Triton CG 50-Dow (5) Neodol 91-8 - Shell (6) Mackam SB 50-Solvay Novacare | | | | |

It can clearly be seen that the antibacterial efficacy of the bispyridinium alkane is superior to that of the biocidal quaternary ammonium compound in all the exemplified compositions as demonstrated by the much lower minimal biocidal concentration of the bispyridinium alkane octenidine dihydrochloride compared to that of the biocidal quaternary ammonium compound BTC®2125. The data provided indicates that octenidine dihydrochloride can be used at much lower concentrations than other commonly used antimicrobial actives, such as quaternary ammonium compounds, to provide acidic compositions with very potent antimicrobial efficacy.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An antimicrobial cleaning composition having a pH of from about 2 to about 4 wherein the composition comprises a bispyridinium alkane antimicrobial active, a surfactant and an acid.

2. A composition according to claim 1 wherein the composition is substantially free of a quaternary ammonium antimicrobial active.

3. A composition according to any of claims 1 or 2 wherein the composition is an aqueous liquid.

4. A composition according to any of the preceding claims wherein the bispyridinium alkane antimicrobial active is octenidine dihydrochloride.

5. A composition according to any of the preceding claims comprising from about 0.0025 to about 0.5% by weight of the composition of the bispyridinium alkane antimicrobial active.

6. A composition according to any of the preceding claims comprising from about 0.0025% to about 1% by weight of the composition of an acid selected from the group consisting of formic acid, acetic acid, benzoic acid, malonic acid, citric acid, maleic acid, fumaric acid, succinic acid, gluconic acid, glutaric acid, lactic acid, cinnamic acid, salicylic acid, oxalic acid, itaconic acid, glycolic acid, adipic acid and mixtures thereof.

7. A composition according to any of the preceding claims comprising from 0.05 to 7% by weight of the composition of a surfactant wherein the surfactant is selected from the group consisting of alkyl polyglucoside, alkyl alkoxylate, betaine, sultaine, alkyl glucamine, alkyl glucamide, alkyl amine oxide and mixtures thereof.

8. A composition according to the preceding claim wherein the surfactant is selected from the group consisting of D-glucopyranose oligomeric decyl octyl glucoside; linear alcohol alkoxylate preferably C₈-C₁₆ alkyl alkoxylate with eight or less ethoxylate units; branched alcohol alkoxylate, preferably 2-propylheptyl alcohol alkoxylate and 2-ethylhexyl alcohol alkoxylate; N,N-dimethyl-N-dodecylglycine betaine; lauramidopropyl betaine; alkyl amidopropyl hydroxysultaine; N-decanoyl-N-methylglucamine; octyl dimethyl amine oxide;, decyl dimethyl amine oxide;, dodecyl dimethyl amine oxide;, tetradecyl dimethyl amine oxide;, C₁₂-₁₈ dihydroxyethyl amine oxide and mixtures thereof.

9. A composition according to the preceding claim wherein the surfactant is selected from the group consisting of D-glucopyranose oligomeric decyl octyl glucoside, C₉ -C₁₁ EO8 linear alkyl ethoxylate, saturated synthetic short chain fatty alcohol with 5 ethoxylate units; 2-propylheptyl EO5 alkylalkoxylate, 2-ethylhexyl PO5EO6 alkylalkoxylate, N,N-dimethyl-N-dodecylglycine betaine, alkyl amidopropyl hydroxysultaine, N-decanoyl-N-methylglucamine, octyl dimethyl amine oxide, decyl dimethyl amine oxide, dodecyl dimethyl amine oxide and mixtures thereof.

10. A composition according to the preceding claim wherein the surfactant is selected from the group consisting of D-glucopyranose oligomeric decyl octyl glucoside, C₉-C₁₁ EO8 linear alkyl ethoxylate; saturated synthetic short chain fatty alcohol with 5 ethoxylate units; 2-ethylhexyl PO5EO6 alkylalkoxylate, N,N-dimethyl-N-dodecylglycine betaine, alkyl amidopropyl hydroxysultaine, decyl dimethyl amine oxide, dodecyl dimethyl amine oxide and mixtures thereof.

11. A composition according to any of the preceding claims comprising from about 0.025% to about 5.0% by weight of the composition of a chelant selected from the group consisting of: amino-carboxylates, preferably diethylenetriaminepentaacetic acid, tetrasodium glutamate diacetate methylglycindiacetic acid polyitaconic acid; phosphonate chelating agents, and mixtures thereof.

12. A composition according to any of the preceding claims further comprising a rheology modifier.

13. A composition according to any of the preceding claims comprising
a) from 0.001 to 0.5% by weight of the composition of octenidine dihydrochloride;
b) from 0.05% to 7%, preferably from 0.05 to 5% by weight of the composition of a surfactant selected from the group consisting of amine oxide surfactant, alkyl alkoxylate surfactant, alkyl polyglucoside surfactant, betaine surfactant, alkyl glucamide surfactant and mixtures thereof; and
c) from 0.0025 to 10% by weight of the composition of an acid.

14. An article treated with a composition according to any of the preceding claims wherein the article is in the form of a disposable or partially reusable substrate comprising one or more nonwoven layers and preferably the substrate has a load factor of from about 3 times to about 10 times of composition per gram of nonwoven substrate.

15. A method of sanitizing a hard surface comprising the step of contacting the surface with a composition according to any of claims 1 to 13 and optionally but preferably wiping the surface with a substrate.

16. A method of sanitizing a fabric comprising the step of contacting the fabric with a composition according to any of claims 1 to 13.
